# EUROPÄISCHE PATENTSCHRIFT

(11) **EP 1 310 172 B1**
(45) Veröffentlichungstag und Bekanntmachung des Hinweises auf die Patenterteilung: **31.08.2005**
(21) Anmeldenummer: 02024989.2
(22) Anmeldetag: 07.11.2002
(51) Int. Cl.: A23K 1/16, C07C 51/10, C07C 51/44

(54) **Verfahren zur Herstellung von Metallformat-Ameisensäure-Mischungen**
Process for the preparation of metalformate-formic acid mixtures
Procédé de préparation de mélanges de formate de métal et d'acide formique

(30) Priorität: 09.11.2001 DE 10154757
(43) Veröffentlichungstag der Anmeldung: 14.05.2003
(73) Patentinhaber: BASF Aktiengesellschaft, 67056 Ludwigshafen (DE)
(72) Erfinder: Ströfer, Eckhard, Dr., 68163 Mannheim (DE); Wostbrock, Karl-Heinz, Dr., 67591 Mörstadt (DE); Weber, Markus, Dr., 67061 Ludwigshafen (DE)
(74) Vertreter: Isenbruck, Günter

(56) Entgegenhaltungen:
- WO-A-96/35657
- US-A- 3 262 973
- US-A- 4 237 318

## Beschreibung

Gegenstand der Erfindung ist ein Verfahren zur Herstellung von Metallformiat-Ameisensäure-Mischungen, wobei die Metallformiate durch Umsetzung von Metallhydroxiden mit Kohlenmonoxid (CO) in Gegenwart eines Katalysators erhalten werden. Edukte sind die Hydroxide von Alkalimetallen und/oder Erdalkalimetallen, insbesondere von Natrium (Na), Kalium (K) und/oder Calcium (Ca).

Metallformiat-Ameisensäure-Mischungen besitzen z. B. große Bedeutung als Dünger und Tierfutterzusatz. Bei der Verwendung als Tierfutterzusatz ist darauf zu achten, dass die Metallformiate mit der Ameisensäure unter Bildung von Disalzen der Ameisensäure reagieren können, so dass dann aus der Mischung keine Ameisensäure freigesetzt wird. Kaliumformiat selbst wird auch als Ölfeldchemikalie (Bohrmittelflüssigkeit) verwendet.

Aus dem Stand der Technik sind bereits Verfahren zur Herstellung von Metallformiaten aus Metallhydroxiden und Kohlenmonoxid bekannt.

Gemäß A.F. Hollemann, N. Wiberg, Lehrbuch der anorganischen Chemie, Walter de Gruyter Verlag Berlin New York, 1985, 91. - 100. Auflage, Seite 722 lässt sich Natriumformiat durch Einleiten von Kohlenmonoxid in Natronlauge bei 150 bis 170°C und einem Druck von 3 bis 4 bar herstellen. Gemäß dem gleichen Lehrbuch Seite 947 wird Kaliumformiat durch Einwirkung von Kohlenmonoxid auf eine wässrige Lösung von Kaliumsulfat und Ätzkalk bei 230°C und 30 bar gewonnen.

In dem Verfahren der US 4,157,246 zur Koks-Verarbeitung wird die Bildung von wasserlöslichen Alkalimetallformiaten dazu verwendet, die Alkalimetalle wie Kalium und Cäsium aus den eingesetzten alkalimetallhaltigen Katalysatoren zurückzugewinnen. Hierzu werden die alkalimetallhaltigen Katalysatorrückstände mit Wasser und Calcium- und/oder Magnesium-Verbindungen wie Calciumhydroxid oder Magnesiumhydroxid versetzt; es wird Kohlenmonoxid eingeleitet - gegebenenfalls unter Zusatz von Alkalimetallsulfat - und man läßt diese Mischung bei ca. 140 bis 390°C unter Bildung von wasserlöslichen Alkalimetallformiaten reagieren. Bei Zusatz von Alkalimetallsulfaten reagieren diese mit Calciumhydroxid zu Calciumsulfat. Da Calciumsulfat aus der Reaktionslösung ausfällt, wird dadurch das Reaktionsgleichgewicht auf die Seite der Produkte verschoben.

Die US 3,262,973 beschreibt ein Verfahren zur Herstellung von Alkalimetall- und Ammoniumformiaten, ausgehend von Alkalimetall- und Ammoniumhydroxid sowie Kohlenmonoxid in einer alkoholischen Lösung, die gegebenenfalls Wasser enthält. Als Alkohole werden beispielsweise C₁-C₄-Alkanole, Cyclohexanol, Furfurylalkohol und Benzylalkohol eingesetzt. Es kann entweder reines Kohlenmonoxid oder ein Gasgemisch enthaltend Kohlenmonoxid eingesetzt werden, so dass das Verfahren auch zur Abtrennung von CO aus Gasgemischen eingesetzt werden kann. Da die Alkalimetall- und Ammoniumformiate - im Gegensatz zu den Alkalimetall- und Ammoniumhydroxiden - in der alkoholischen Lösung nicht löslich sind, bildet sich ein Alkalimetall- bzw. Ammoniumformiatniederschlag, der sich durch Dekantieren, Filtrieren oder Zentrifugieren von der Mutterlauge, die rezykliert werden kann, abtrennen läßt. Das Verfahren kann sowohl diskontinuierlich als auch kontinuierlich durchgeführt werden. Die Reaktionstemperaturen sind in weiten Bereichen variabel. Im allgemeinen liegen sie bei Normaldruck zwischen 0°C und der Siedetemperatur des verwendeten Alkohols. Bevorzugt sind Temperaturen von 10 bis 30°C. In den Beispielen wird die Umsetzung von 8 bis 10 Gew.-%-iger ethanolischer NaOH- bzw. KOH-Lösung beschrieben.

Auch Verfahren zur Herstellung von Formiat-Ameisensäure-Mischungen sind in der Literatur bereits bekannt. Gemäß dem Verfahren der WO 96/35657 werden Kaliumhydroxid, - carbonat, -hydrogencarbonat oder -formiat, Natriumhydroxid, -carbonat, -hydrogencarbonat oder -formiat, Cäsiumhydroxid, -carbonat, -hydrogencarbonat oder -formiat, Ammoniumformiat oder Ammoniak mit Ameisensäure enthaltend 0 bis 50% Wasser bei 40 bis 100°C miteinander vermischt, gekühlt, filtriert und getrocknet, und so werden letztendlich die entsprechenden Disalze der Ameisensäure erhalten. Da jedoch im Falle der Metallhydroxide, -carbonate oder -hydrogencarbonate jeweils 1 mol der Ameisensäure dazu verwendet wird, aus dem Metallsalz das entsprechende Formiat herzustellen, ist dieses Verfahren relativ teuer.

Aufgabe der Erfindung ist es, ein Verfahren bereitzustellen, durch welches Metallformiat-Ameisensäure-Mischungen in industriellem Maßstab kostengünstig hergestellt werden können, wobei die Metallformiate die Formiate von Alkalimetallen und/oder Erdalkalimetallen sind, insbesondere die Formiate von Na, K und/oder Ca.

Die Aufgabe wird erfindungsgemäß gelöst durch ein Verfahren zur Herstellung von Metallformiat-Ameisensäure-Mischungen enthaltend folgende Schritte:
a) Umsetzung von mindestens einem Metallhydroxid mit Kohlenmonoxid (CO) oder einem CO-haltigen Gas in Gegenwart eines Katalysators in einer wasserhaltigen Lösung zu dem oder den entsprechenden Metallformiat(en);
b) destillative Abtrennung des Wassers und des Katalysators;
c) Zumischen von Ameisensäure;
d) gegebenenfalls Einstellen der Temperatur der nach den Schritten a) bis c) erhaltenen Metallformiat-Ameisensäure-Mischung auf Lagertemperatur;
wobei in Schritt a) als Metallhydroxide die Hydroxide von Alkalimetallen und/oder Erdalkalimetallen eingesetzt werden und Schritt c) auch zeitlich vor Schritt b) durchgeführt werden kann.

Das erfindungsgemäße Verfahren erlaubt die kostengünstige Herstellung von Metallformiat-Ameisensäure-Mischungen in industriellem Maßstab.

Die Verfahrensschritte a) bis d) werden nun im einzelnen näher erläutert.

### Schritt a)

Die Umsetzung der Hydroxide von Alkalimetallen und/oder Erdalkalimetallen mit CO oder einem CO-haltigen Gas in Gegenwart eines Katalysators in einer wasserhaltigen Lösung zu dem oder den entsprechenden Metallformiat(en) erfolgt im allgemeinen bei Temperaturen von 20 bis 250°C, bevorzugt von 30 bis 160°C, besonders bevorzugt von 90 bis 120°C, und Drücken von 1 bis 50 bar, bevorzugt von 3 bis 40 bar, besonders bevorzugt von 8 bis 20 bar.

Im Vergleich zu sonst üblichen Verfahren zur Formiat-Herstellung kann in höheren Konzentrationsbereichen des Metallhydroxids, und tendenziell bei höheren Drücken und niedrigeren Temperaturen gearbeitet werden. Da die Reaktion durch den Stoffübergang limitiert ist, lassen sich durch gute Durchmischung, beispielsweise unter Einsatz von Mischdüsen, höhere Raum-Zeit-Ausbeuten erzielen.

Die Umsetzung kann sowohl kontinuierlich als auch diskontinuierlich erfolgen. Bevorzugt ist die kontinuierliche Umsetzung. Im allgemeinen wird die Reaktion so geführt, dass das Metallhydroxid nahezu quantitativ zu Metallformiat umgesetzt wird. Das Ende der Reaktion läßt sich durch Bestimmung des Anteils an Metallhydroxid in der Lösung, beispielsweise durch Titration, bestimmen. Die Reaktion wird vorteilhafterweise so lange geführt, bis der Anteil des Metallhydroxids in der Reaktionslösung unter 0,1 Gew.-%, bevorzugt unter 0,04 Gew.-%, besonders bevorzugt unter 0,01 Gew.-% liegt.

Die Umsetzung kann prinzipiell in jeder Art von Reaktionsgefäß durchgeführt werden. Bevorzugt wird sie in einem Rührkessel mit Begasungseinrichtung, in einer Blasensäule oder einem Schlaufenreaktor durchgeführt. Besonders bevorzugt erfolgt die Umsetzung in einem Schlaufenreaktor oder einer Blasensäule, ganz besonders bevorzugt in einem Schlaufenreaktor, da hier aufgrund der großen Phasengrenzfläche zwischen der Metallhydroxid und Katalysator enthaltenden wasserhaltigen Lösung und dem eingebrachten CO oder dem CO-haltigen Gas eine hohe Absorptions- und damit auch eine hohe Reaktionsgeschwindigkeit resultiert.

Die Metallhydroxide werden in der Regel als wäßrige Lösung eingesetzt. Die Konzentration dieser Metallhydroxid-Lösungen beträgt im allgemeinen 25 bis 50 Gew.-%, bevorzugt 45 bis 50 Gew.-%, besonders bevorzugt 48,5 bis 50 Gew.-%. Die wäßrige Lösung kann auch mehrere Metallhydroxide enthalten. An die Reinheit der eingesetzten Metallhydroxid-Lösungen werden im allgemeinen keine besonderen Anforderungen gestellt. Im allgemeinen werden daher technische Metallhydroxid-Lösungen verwendet. Diese können beispielsweise mit einem Gehalt an Natriumionen und Carbonationen < 0,1 Gew.%, oder einem Gehalt an Chloridionen und Sulfationen < 25 mg/kg Metallhydroxid verunreinigt sein. Das erfindungsgemäße Verfahren läßt sich auch mit reinen Metallhydroxid-Lösungen durchführen. Bevorzugt sind die Hydroxide von Natrium, Kalium und/oder Calcium, besonders bevorzugt ist Kaliumhydroxid.

CO kann sowohl als Einzelkomponente, als auch im Gemisch mit anderen Gasen eingesetzt werden. Solche anderen Gase sind unter den Reaktionsbedingungen inerte Gase, beispielsweise N₂ und die Edelgase.

Wird CO im Gemisch mit anderen Gasen eingesetzt, so beträgt der Anteil des CO im Gasgemisch mindestens 5 Vol-%, bevorzugt mindestens 10 Vol-%, besonders bevorzugt mindestens 25 Vol-%, ganz besonders bevorzugt mindestens 50 Vol-%. Der CO-Partialdruck bei der Umsetzung beträgt im allgemeinen 1 bis 60 bar, bevorzugt 20 bis 55 bar, besonders bevorzugt 40 bis 55 bar.

An die Reinheit des verwendeten CO oder eines entsprechenden CO-haltigen Gasgemisches werden im allgemeinen keine besonderen Anforderungen gestellt. Die Reaktion läßt sich daher sowohl mit reinem als auch mit technischem CO oder den entsprechenden CO-haltigen Gasgemischen durchführen. Bevorzugt wird reines CO oder ein CO-haltiges Gasgemisch mit einem CO-Gehalt von mindestens 50 Vol-% eingesetzt, besonders bevorzugt wird reines CO verwendet.

CO oder das CO-haltige Gasgemisch kann sowohl von oben als auch von unten in den Reaktor, also im Gleichstrom oder Gegenstrom, eingespeist werden. Es ist auch möglich, CO direkt in die Metallhydroxid- oder Katalysator-Leitung einzudüsen sowie CO zwischeneinzuspeisen, also über eine Seitenzufuhr.

Als Katalysator wird mindestens ein Katalysator aus der Gruppe Alkohol und Ameisensäureester eingesetzt. Prinzipiell sind alle Katalysatoren geeignet, in denen sich die Metallhydroxide gut lösen. Geeignete Katalysatoren sind beispielsweise gesättigte lineare C₁-C₄-Alkanole, ungesättigte lineare C₃-C₄-Alkanole, gesättigte verzweigte C₃-C₄-Alkanole und ungesättigte verzweigte C₄-Alkanole, sowie deren Ameisensäureester. Wird ein Alkohol im Gemisch mit einem Ameisensäureester als Katalysator eingesetzt, so wird meist der Ameisensäureester dieses Alkohols eingesetzt. Bevorzugt werden gesättigte lineare C₁-C₄-Alkanole und gesättigte verzweigte C₃-C₄-Alkanole, besonders bevorzugt wird Methanol verwendet. Der Katalysator wird im allgemeinen in einer Konzentration von 2 bis 40 Gew.-%, bevorzugt von 10 bis 25 Gew.-%, besonders bevorzugt von 15 bis 20 Gew.-%, - bezogen auf die gesamte Reaktionslösung - eingesetzt.

An die Reinheit des Katalysators werden - wie beim Metallhydroxid und dem CO oder CO-haltigen Gasgemisch - ebenfalls keine besonderen Anforderungen gestellt. Im allgemeinen werden daher technische Alkohole und/oder deren Ameisensäureester eingesetzt.

Die nach Schritt a) erhaltenen Metallformiate liegen im allgemeinen in einer Konzentration von 10 bis 90 Gew.-%, bevorzugt von 30 bis 80 Gew.-%, besonders bevorzugt von 40 bis 70 Gew.-%, in der Reaktionslösung vor.

Durch die Reaktionsführung ist gewährleistet, daß es nicht zum Auskristallisieren oder Ausfällen von Metallformiat kommt, so daß die Reaktionsmischung während der Dauer der Reaktion als Lösung vorliegt. Ausgefällte Substanzen und hierdurch verursachte Verstopfungen in Leitungen können sonst zu teuren und zeitaufwendigen Betriebsausfällen führen. Dies wird im erfindungsgemäßen Verfahren vermieden.

### Schritt b)

Zur Gewinnung des Metallformiats werden Wasser und Katalysator aus der Reaktionslösung entfernt. Da Wasser und der Katalysator die niedrigsten Siedepunkte in der durch Schritt a) erhaltenen Reaktionslösung haben, lassen sie sich einfach durch Abdestillieren abtrennen, wobei die Destillation in einer beliebig gestalteten Destillationskolonne erfolgen kann, in die die Reaktionsmischung nach Beendigung von Schritt a) überführt wird. Es kann auch mehr als eine Destillationskolonne eingesetzt werden. In diesem Fall sind die Destillationskolonnen miteinander verschaltet.

Wasser und Katalysator lassen sich sowohl gleichzeitig, unter Umständen als Azeotrop, als auch nacheinander abtrennen.

In einer Ausführungsvariante werden der Katalysator und das Wasser über Kopf einer ersten Destillationskolonne gemeinsam abgetrennt und in einer weiteren Kolonne weiter aufgetrennt. Die Metallformiatschmelze wird über den Sumpf der ersten Destillationskolonne abgezogen.

In einer anderen Ausführungsvariante wird der Katalysator über Kopf der Destillationskolonne und Wasser und Metallformiat werden über den Sumpf der Destillationskolonne abgezogen und in einer zweiten Kolonne voneinander getrennt.

In einer dritten Variante wird eine Trennwandkolonne eingesetzt. Hier können Katalysator, Metallformiat und Wasser in einem einzigen Schritt voneinander getrennt werden. Die Verwendung einer Trennwandkolonne ist bevorzugt, da sie besonders kostengünstig ist.

In allen drei Varianten fällt das Metallformiat im Sumpf einer Destillationskolonne als Metallformiat-Schmelze an. Der Wasser-Anteil der Metallformiat-Schmelze liegt im allgemeinen unter 2 Gew.%, bevorzugt unter 0,5 Gew. %, besonders bevorzugt < 0,1 Gew. %.

Der Katalysator wird bevorzugt, gegebenenfalls als wäßrige Lösung, ganz oder teilweise dem Reaktor rückgeführt. Das Wasser kann ganz oder teilweise zur Herstellung einer Lösung des Metallhydroxids wiederverwendet oder anderen Behandlungseinrichtungen zugeführt werden.

Die Destillation wird im allgemeinen bei einem Druck von 0,2 bis 15 bar, bevorzugt von 1 bis 5 bar, besonders bevorzugt von 1 bis 3 bar, durchgeführt. Die Destillationstemperatur liegt im allgemeinen bei 50 bis 220°C, bevorzugt bei 60 bis 150°C, besonders bevorzugt bei 90 bis 120°C.

### Schritt c)

Die im Sumpf der Destillationskolonne angefallene Metallformiat-Schmelze, die im allgemeinen unter Normaldruck eine Temperatur von 100 bis 255°C, bevorzugt eine Temperatur von 120 bis 180°C, besonders bevorzugt von 140 bis 160°C, hat, wird nun in einen beliebig gestalteten Mischer überführt, gegebenenfalls auf eine Temperatur von 110 bis 120°C, bevorzugt auf eine Temperatur von 115°C bis 120°C, gebracht und im Mischer mit Ameisensäure bei Temperaturen von 8°C bis 120°C, bevorzugt bei Temperaturen von 50°C bis 120°C, besonders bevorzugt bei Temperaturen von 90°C bis 120°C, ganz besonders bevorzugt bei Temperaturen von 100°C bis 120°C, vermischt. Die Obergrenze für die Temperatur ergibt sich durch die Zersetzungstemperatur von Ameisensäure von 120°C, die Untergrenze durch den Schmelzpunkt der Ameisensäure von 8°C. Der Druck während des Mischvorgangs beträgt im allgemeinen von 0,5 bis 10 bar, bevorzugt von 1 bis 5 bar, besonders bevorzugt von 1 bis 2 bar.

Die eingesetzte Ameisensäure liegt im allgemeinen als wäßrige Lösung mit einer Konzentration von 80 bis 99,9 Gew.-%, bevorzugt von 94 bis 99,9 Gew.-%, besonders bevorzugt von 98 bis 99,99 Gew.-%, vor. Technische Reinheit ist ausreichend; reine (wäßrige) Ameisensäure läßt sich jedoch auch einsetzen.

Da Ameisensäure korrosiv ist, sollte der Mischer aus einem ameisensäureresistenten Material bestehen, zum Beispiel aus austenitischem Chrom-Nickel-Stahl oder hochlegierten Edelstählen. Die hochlegierten Edelstähle sind bevorzugt.

Als Mischer lassen sich beliebige Rührkessel, Extruder oder statische Mischer - mit oder ohne Wärmetauscheinrichtungen - einsetzen, wobei die Durchmischung mit Hilfe von Reaktionsmischpumpen, Mischdüsen oder Wirbelbetten erfolgt. Bevorzugt werden statische Mischer - mit oder ohne Wärmetauscheinrichtungen - eingesetzt.

Bei Einsatz von Kaliumformiat und/oder Natriumformiat kann der Mischvorgang auch nach den in der WO 96/35657 beschriebenen Methoden erfolgen.

Durch die Mischung der Metallformiate mit der Ameisensäure werden Disalze der Ameisensäure erhalten.

In dem erfindungsgemäßen Verfahren lässt sich Schritt c) auch zeitlich vor Schritt b) durchführen. Die zeitliche Abfolge a), b), c) ist jedoch bevorzugt.

Die Ameisensäure-Konzentration, die Temperatur und der Druck während des Mischvorgangs sind unabhängig davon, ob Schritt c) vor oder nach Schritt b) durchgeführt wird.

Bei der Durchführung des erfindungsgemäßen Verfahrens in der Schrittfolge a), c), b) wird die dem Reaktor nach Durchführung von Schritt a) entnommene Reaktionslösung einem Mischer zugeführt, gegebenenfalls auf Temperaturen von 110 bis 120°C, bevorzugt auf Temperaturen von 115 bis 120°C, gebracht und in einem der obenbeschriebenen Mischer mit Ameisensäure vermischt. Die Temperatur während des Mischvorgangs beträgt im allgemeinen von 8 bis 120°C, bevorzugt von 50 bis 120°C, besonders bevorzugt von 90 bis 120°C, ganz besonders bevorzugt von 100 bis 120°C. Der Druck liegt bei 0,5 bis 10 bar, bevorzugt bei 1 bis 5 bar, besonders bevorzugt bei 1 bis 2 bar.

Diese Reaktionsmischung wird anschließend einer Trenneinheit zugeführt, in der Wasser und Katalysator sowie in der Ameisensäure gegebenenfalls vorhandene leichtsiedende Verunreinigungen wie Formaldehyd abdestilliert werden. Der Katalysator wird bevorzugt als wäßrige Lösung ganz oder teilweise dem Reaktor rückgeführt. Das Wasser läßt sich ganz oder teilweise zur Herstellung von Metallhydroxid-Lösungen wiederverwenden. Die abgetrennten Verunreinigungen der Ameisensäure werden verworfen.

Da in dieser Reaktionsmischung jetzt Ameisensäure enthalten ist, ist darauf zu achten, dass die Trenneinheit ebenfalls eines der obengenannten ameisensäureresistenten Materialien enthält. Als Trenneinheit kommt jede beliebige Destillationskolonne in Frage. Es kann jedoch auch mehr als eine Destillationskolonne eingesetzt werden. In diesem Fall sind die Destillationskolonnen miteinander verschaltet. Bevorzugt wird eine Trennwandkolonne verwendet, die besonders kostengünstig ist.

### Schritt d)

Die Temperatur der nach den Schritten a) bis c) erhaltenen Metällformiat-Ameisensäure-Mischungen wird auf Lagertemperatur eingestellt. Unter Lagertemperatur versteht man im Rahmen dieser Erfindung Temperaturen von 0 bis 40°C, bevorzugt von 20 bis 30 °C, besonders bevorzugt von 20 bis 25°C. Als Wärmetauscher sind alle gängigen, kommerziell erhältlichen Wärmetauscher geeignet; beispielsweise wird ein Wirbelbett eingesetzt.

Die so auf Lagertemperatur gebrachten Metallformiat-Ameisensäure-Mischungen werden anschließend dem Lager oder Versand in fester Form, d. h. beispielsweise als Kristalle oder Granulate, zugeführt.

Gegebenenfalls erfolgt hierbei eine Trocknung gemäß der in der WO 96/35657 beschriebenen Vorgehensweise, wobei auch Trockenmittel zu der Metallformiat-Ameisensäure-Mischung zugegeben werden kann. Bisweilen wird der Mischung auch Anti-Backmittel (anti-caking agent) zugegeben.

Der Restgehalt an Wasser in der Metallformiat-Ameisensäure-Mischung liegt im allgemeinen unter 0,5 Gew.%.

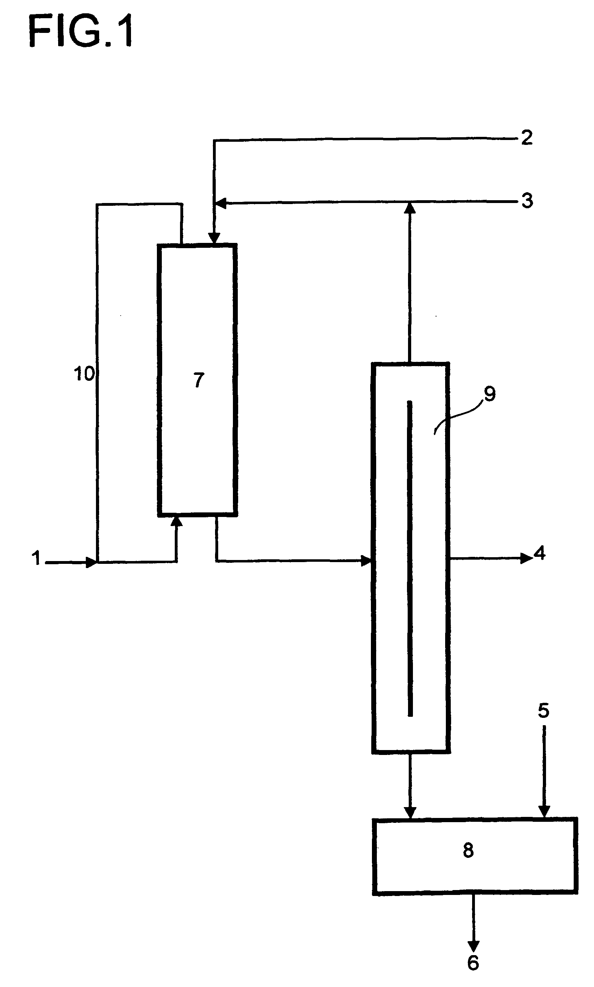

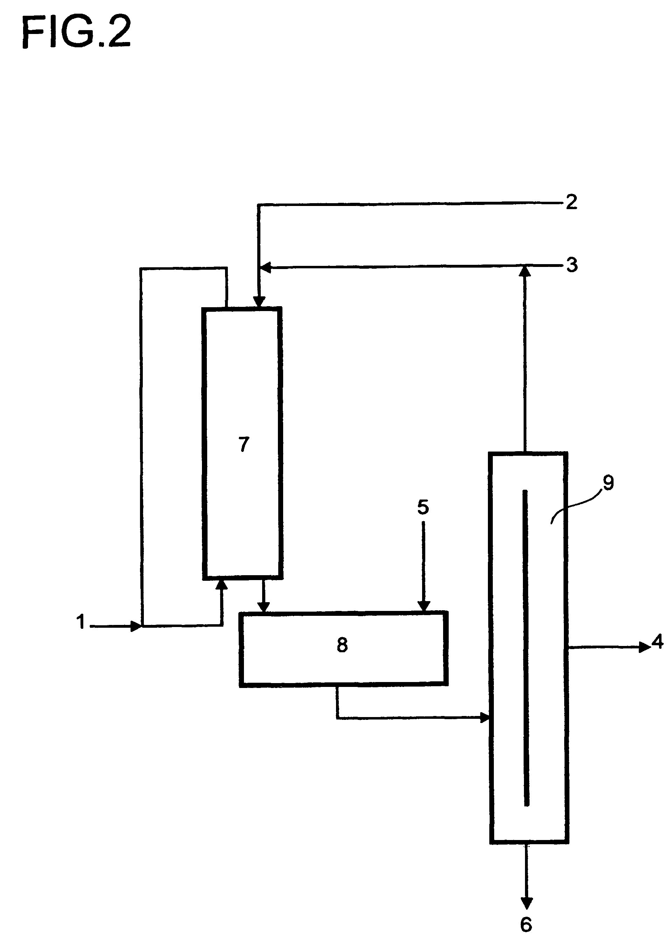

Die anliegende Zeichnung zeigt in den Figuren 1 und 2 schematisch Anlagen, in denen das erfindungsgemäße Verfahren bevorzugt durchgeführt wird, nämlich in
- **Fig. 1**: eine schematische Darstellung einer Anlage zur Herstellung von Metallformiat-Ameisensäure-Mischungen für den kontinuierlichen Betrieb, wobei Schritt c) nach Schritt b) durchgeführt wird.
- **Fig. 2**: eine schematische Darstellung einer Anlage zur Herstellung von Metallformiat-Ameisensäure-Mischungen für den kontinuierlichen Betrieb, wobei Schritt c) vor Schritt b) durchgeführt wird.

In einer Anlage gemäß Figur 1 werden die einzelnen Verfahrensschritte in der zeitlichen Reihenfolge a), b) und c) durchgeführt. CO oder ein CO-haltiges Gasgemisch wird über einen Einlass 1 von unten im Gegenstrom in einen Reaktor 7 geführt und nicht umgesetztes CO wird von oben dem Reaktor entnommen und ganz oder teilweise über eine Leitung 10 wieder in den Reaktor 7 rückgeführt. Ein Katalysatorstrom wird über eine Zuleitung 3 einer wäßrigen Metallhydroxid-Lösung in einer Zuleitung 2 zugemischt und von oben in den Reaktor 7 geführt. Die Reaktion wird im allgemeinen bei einer Temperatur von 20 bis 250°C, bevorzugt von 30 bis 160°C, besonders bevorzugt von 90 bis 120°C, durchgeführt. Der CO-Partialdruck liegt im allgemeinen bei 1 bis 60 bar, bevorzugt bei 20 bis 55 bar, besonders bevorzugt bei 40 bis 55 bar. Bevorzugt wird als Reaktor 7 ein Schlaufenreaktor eingesetzt.

Ein Teil der Reaktionslösung wird dem Reaktor 7 kontinuierlich entnommen und in eine Trenneinheit 9 überführt. Wird als Trenneinheit 9 eine Destillationskolonne eingesetzt, so wird der Katalysator über Kopf abgezogen und ganz oder teilweise dem Reaktor 7 rückgeführt. Wasser wird über die Ableitung 4 der Trenneinheit 9 entnommen. Diese Ableitung stellt entweder einen Seitenabzug einer Trennwandkolonne oder den Sumpfabzug der oberen Kolonne bei einer Verschaltung von 2 Kolonnen dar. Das Wertprodukt Metallformiat fällt am Sumpf der Kolonne als Schmelze an und wird von dort entnommen und in einen Mischer 8 überführt. Über eine Zuleitung 5 wird dem Mischer 8 kontinuierlich Ameisensäure mit einer Konzentration von ≥ 80 Gew.-%, bevorzugt ≥ 94 Gew.-%. besonders bevorzugt ≥ 98 Gew.-%, zugegeben. Das Wertprodukt, die Metallformiat-Ameisensäure-Mischung, wird über eine Ableitung 6 dem Mischer 8 entnommen. Während in der Destillationskolonne 9 im allgemeinen Temperaturen von 50 bis 220°C, bevorzugt von 60 bis 150°C, besonders bevorzugt von 90 bis 120°C und Drücke von 0,2 bis 15 bar, bevorzugt von 1 bis 5 bar, besonders bevorzugt von 1 bis 3 bar herrschen, beträgt die Temperatur im Mischer 8 von 8 bis 120°C, bevorzugt von 50 bis 120°C, besonders bevorzugt von 90 bis 120°C, ganz besonders bevorzugt von 100 bis 120°C, und der Druck von 0,5 bis 10 bar, bevorzugt von 1 bis 5 bar, besonders bevorzugt von 1 bis 2 bar.

Anschließend wird die Metallformiat-Ameisensäure-Mischung über einen Wärmetauscher auf eine Lagertemperatur von 0 bis 40°C, bevorzugt von 20 bis 30°C, besonders bevorzugt von 20 bis 25°C, gebracht und dem Lager oder Versand in fester Form zugeführt (nicht dargestellt). Als Wärmetauscher wird beispielsweise ein Wirbelbett eingesetzt.

Wird das erfindungsgemäße Verfahren in einer Anlage gemäß Figur 2 durchgeführt, so werden die einzelnen Verfahrensschritte in der zeitlichen Reihenfolge a), c) und b) durchgeführt. CO oder ein CO-haltiges Gasgemisch werden über einen Einlass 1 von unten in einen Reaktor 7 geführt und nicht umgesetztes CO wird von oben dem Reaktor entnommen und ganz oder teilweise über eine Leitung 10 wieder in den Reaktor 7 rückgeführt. Eine wäßrige Metallhydroxid-Lösung, der ein Katalysatorstrom über eine Zuleitung 3 zugemischt wird, wird über eine Zuleitung 2 von oben dem Reaktor 7 zugeführt. Bevorzugt wird als Reaktor 7 ein Schlaufenreaktor eingesetzt. Der CO-Partialdruck liegt während der Umsetzung im allgemeinen bei 1 bis 60 bar, bevorzugt bei 20 bis 55, besonders bevorzugt bei 40 bis 55 bar. Die Temperaturen im Reaktor 7 betragen im allgemeinen 20 bis 250°C, bevorzugt 30 bis 160°C, besonders bevorzugt 90 bis 120°C, und die Drücke von 1 bis 50 bar, bevorzugt von 3 bis 40 bar, besonders bevorzugt von 8 bis 20 bar. Dem Reaktor 7 wird kontinuierlich ein Teil der Reaktionslösung entnommen und in einen Mischer 8 überführt. Über eine Zuleitung 5 wird Ameisensäure in den Mischer 8 geleitet, wobei die Ameisensäure in einer Konzentration von ≥ 80 Gew.-%, bevorzugt ≥ 94 Gew.-%. besonders bevorzugt ≥ 98 Gew.-%, wäßrige Lösung vorliegt.

Die Temperatur während des Mischvorgangs im Mischer 8 beträgt im allgemeinen von 8 bis 120°C, bevorzugt von 50 bis 120°C, besonders bevorzugt von 90 bis 120°C, ganz besonders bevorzugt von 100 bis 120°C, und der Druck 0,5 bis 10 bar, bevorzugt von 1 bis 5 bar, besonders bevorzugt 1 bis 2 bar. Die dadurch erhaltene Mischung wird kontinuierlich in eine Destillationskolonne 9, die aus ameisensäureresistentem Material besteht, überführt. Die Destillation wird im allgemeinen bei Temperaturen von 50 bis 140°C, bevorzugt von 80 bis 120°C, besonders bevorzugt von 90 bis 120°C, durchgeführt. Der Druck in der Destillationskolonne 9 liegt im allgemeinen bei 0,2 bis 15 bar, bevorzugt bei 1 bis 5 bar, besonders bevorzugt bei 1 bis 3 bar. Der Katalysator wird kontinuierlich über den Kopf der Destillationskolonne abgezogen und bevorzugt ganz oder teilweise dem Reaktor 7 rückgeführt. Das Wasser wird dem Seitenteil der Destillationskolonne 4 entnommen und entweder ganz oder teilweise zur Herstellung wäßriger Metallhydroxid-Lösungen wiederverwendet oder ganz oder teilweise anderen Behandlungseinrichtungen zugeführt. Am Sumpf der Kolonne fällt die gewünschte Metallformiat-Ameisensäure-Mischung an.

Diese wird anschließend über einen Wärmetauscher auf eine Lagertemperatur von 0 bis 40°C, bevorzugt von 20 bis 30°C, besonders bevorzugt von 20 bis 25°C, gebracht und dem Lager oder Versand in fester Form zugeführt. Als Wärmetauscher wird beispielsweise ein Wirbelbett eingesetzt.

### Bezugszeichenliste

In den Figuren 1 und 2 haben die Bezugszeichen folgende Bedeutung:
- 1: Einlass für CO;
- 2: Zuleitung für die wäßrige Metallhydroxid-Lösung;
- 3: Zuleitung für den Katalysator;
- 4: Ableitung für Wasser;
- 5: Zuleitung für die Ameisensäure;
- 6: Ableitung für das Produkt;
- 7: Reaktor;
- 8: Mischer;
- 9: Trenneinheit;
- 10: Leitung.

## Patentansprüche

1. Verfahren zur Herstellung von Metallformiat-Ameisensäure-Mischungen enthaltend folgende Schritte:
a) Umsetzung von mindestens einem Metallhydroxid mit Kohlenmonoxid (CO) oder einem CO-haltigen Gas in Gegenwart eines Katalysators in einer wasserhaltigen Lösung zu dem oder den entsprechenden Metallformiat(en);
b) destillative Abtrennung des Wassers und des Katalysators;
c) Zumischen von Ameisensäure;
d) gegebenenfalls Einstellen der Temperatur der nach den Schritten a) bis c) erhaltenen Metallformiat-Ameisensäure-Mischung auf Lagertemperatur;
wobei in Schritt a) als Metallhydroxide die Hydroxide von Alkalimetallen und/oder Erdalkalimetallen eingesetzt werden und Schritt c) auch zeitlich vor Schritt b) durchgeführt werden kann.

2. Verfahren gemäß Anspruch 1, **dadurch gekennzeichnet, dass** als Metallhydroxide die Hydroxide von Natrium, Kalium und/oder Calcium eingesetzt werden.

3. Verfahren gemäß Anspruch 1 oder 2, **dadurch gekennzeichnet, dass** als Katalysator in Schritt a) mindestens ein Katalysator aus der Gruppe Alkohol und Ameisensäureester eingesetzt wird.

4. Verfahren gemäß Anspruch 3, **dadurch gekennzeichnet, dass** der Katalysator mindestens ein Katalysator aus der Gruppe linearer C₁-C₄-, verzweigter C₃-C₄-Alkanol und deren Ameisensäureester ist.

5. Verfahren gemäß Anspruch 4, **dadurch gekennzeichnet, dass** der Katalysator Methanol ist.

6. Verfahren nach einem der Ansprüche 1 bis 5, **dadurch gekennzeichnet, dass** die Umsetzung in Schritt a) in einem Rührkessel mit Begasungseinrichtung, in einer Blasensäule oder einem Schlaufenreaktor durchgeführt wird.

7. Verfahren nach einem der Ansprüche 1 bis 6, **dadurch gekennzeichnet, dass** das Zumischen in Schritt c) in einem Rührkessel, Extruder oder Mischer - mit oder ohne Wärmetauscheinrichtungen - erfolgt und der Mischvorgang mit Hilfe einer Reaktionsmischpumpe, einer Mischdüse oder eines Wirbelbettes erfolgt.

8. Verfahren nach einem der Ansprüche 1 bis 7, **dadurch gekennzeichnet, dass** Schritt a) bei Temperaturen von 20 bis 250°C und einem Druck von 1 bis 50 bar durchgeführt wird.

9. Verfahren nach einem der Ansprüche 1 bis 8, **dadurch gekennzeichnet, dass** Schritt b) bei Temperaturen von 50 bis 220°C und einem Druck von 0,2 bis 15 bar durchgeführt wird.

10. Verfahren nach einem der Ansprüche 1 bis 9, **dadurch gekennzeichnet, dass** Schritt c) bei Temperaturen von 8 bis 120°C und einem Druck von 0,5 bis 10 bar durchgeführt wird, und/oder dass die Lagertemperatur in Schritt d) eine Temperatur von 0 bis 40°C ist.

## Claims

1. A process for preparing metal formate/formic acid mixtures comprising the following steps:
a) Reacting at least one metal hydroxide with carbon monoxide (CO) or a CO-containing gas in the presence of a catalyst in an aqueous solution to give the corresponding metal formate(s);
b) separating off the water and catalyst by distillation;
c) adding formic acid;
d) if appropriate, adjusting to storage temperature the temperature of the metal formate/formic acid mixture obtained using steps a) to c);
with, in step a), the metal hydroxides being the hydroxides of alkali metals and/or alkaline earth metals and step c) also being able to be carried out in time before step b).

2. A process as claimed in claim 1, wherein the metal hydroxides are the hydroxides of sodium, potassium and/or calcium.

3. A process as claimed in claim 1 or 2, wherein the catalyst in step a) is at least one catalyst selected from the group consisting of alcohol and formic esters.

4. A process as claimed in claim 3, wherein the catalyst is at least one catalyst selected from the group consisting of unbranched C1-C4alkanol, branched C3-C4alkanol and formic esters thereof.

5. A process as claimed in claim 4, wherein the catalyst is methanol.

6. A process as claimed in one of claims 1 to 5, wherein the reaction in step a) is carried out in a stirred tank having a gas-introduction device, in a bubble column, or a loop reactor.

7. A process as claimed in one of claims 1 to 6, wherein the addition in step c) is performed in a stirred tank, extruder or mixer, with or without heat-exchange devices, and the mixing operation is performed using a reaction mixing pump, a mixing nozzle, or a fluidized bed.

8. A process as claimed in one of claims 1 to 7, wherein step a) is carried out at temperatures of from 20 to 250°C and a pressure of from 1 to 50 bar.

9. A process as claimed in one of claims 1 to 8, wherein step b) is carried out at temperatures of from 50 to 220°C and a pressure of from 0.2 to 15 bar.

10. A process as claimed in one of claims 1 to 9, wherein step c) is carried out at temperatures of from 8 to 120°C and a pressure of from 0.5 to 10 bar, and/or wherein the storage temperature in step d) is a temperature from 0 to 40°C.

## Revendications

1. Procédé de préparation de mélanges de formiate métallique-acide formique, comprenant les étapes suivantes :
a) une réaction d'au moins un hydroxyde métallique avec du monoxyde de carbone (CO) ou un gaz contenant du CO en présence d'un catalyseur dans une solution contenant de l'eau pour former le ou les formiates métalliques correspondants,
b) une séparation par distillation de l'eau et du catalyseur,
c) un mélange d'acide formique,
d) éventuellement un ajustement de la température du mélange de formiate métallique-acide formique obtenu selon les étapes a) à c) à une température d'entreposage,
dans lequel, dans l'étape a), on met en oeuvre comme hydroxydes métalliques les hydroxydes de métaux alcalins et/ou de métaux alcalino-terreux et on peut aussi effectuer l'étape c) dans le temps avant l'étape b).

2. Procédé suivant la revendication 1, **caractérisé en ce que**, comme hydroxydes métalliques, on met en oeuvre les hydroxydes de sodium, de potassium et/ou de calcium.

3. Procédé suivant la revendication 1 ou 2, **caractérisé en ce que**, comme catalyseur dans l'étape a), on met en oeuvre au moins un catalyseur du groupe des alcools et des esters d'acide formique.

4. Procédé suivant la revendication 3, **caractérisé en ce que** le catalyseur est au moins un catalyseur du groupe des alcanols en C₁-C₄ linéaires, des alcanols en C₃-C₄ ramifiés et de leurs esters d'acide formique.

5. Procédé suivant la revendication 4, **caractérisé en ce que** le catalyseur est du méthanol.

6. Procédé suivant l'une des revendications 1 à 5, **caractérisé en ce que** la réaction dans l'étape a) est effectuée dans une cuve d'agitation munie d'un dispositif d'alimentation en gaz, dans une colonne à bulles ou dans un réacteur à écoulement en boucles.

7. Procédé suivant l'une des revendications 1 à 6, **caractérisé en ce que** le mélange de l'étape c) a lieu dans une cuve d'agitation, une extrudeuse ou un mélangeur - avec ou sans dispositifs d'échange thermique - et **en ce que** le processus de mélange a lieu à l'aide d'une pompe mélangeuse réactionnelle, d'une tuyère mélangeuse ou d'un lit tourbillonnaire.

8. Procédé suivant l'une des revendications 1 à 7, **caractérisé en ce que** l'étape a) est effectuée à des températures de 20 à 250°C et à une pression de 1 à 50 bars.

9. Procédé suivant l'une des revendications 1 à 8, **caractérisé en ce que** l'étape b) est effectuée à des températures de 50 à 220°C et à une pression de 0,2 à 15 bars.

10. Procédé suivant l'une des revendications 1 à 9, **caractérisé en ce que** l'étape c) est effectuée à des températures de 8 à 120°C et à une pression de 0,5 à 10 bars et/ou **en ce que** la température d'entreposage de l'étape d) est une température de 0 à 40°C.
